# EUROPEAN PATENT SPECIFICATION

(11) **EP 1 126 830 B1**
(45) Date of publication and mention of the grant of the patent: **23.07.2008**
(21) Application number: 99971706.9
(22) Date of filing: 28.10.1999
(51) Int. Cl.: A61K 9/70

(54) **PHARMACEUTICAL PREPARATIONS FOR EXTERNAL USE CONTAINING NON-STEROIDAL ANTI-INFLAMMATORY AND ANALGESIC AGENTS**
PHARMAZEUTISCHE ZUBEREITUNGEN ZUR EXTERNEN ANWENDUNG, DIE NICHT STEROIDALE ANTIINFLAMMATORISCHE UND ANALGETISCHE WIRKSTOFFE ENTHALTEN
PREPARATIONS PHARMACEUTIQUES A USAGE EXTERNE CONTENANT DES AGENTS ANTI-INFLAMMATOIRES ET ANALGESIQUES NON-STEROIDIENS

(30) Priority: 06.11.1998 JP 31573998
(43) Date of publication of application: 29.08.2001
(73) Proprietor: Novartis AG, 4056 Basel (CH)
(72) Inventor: KANEKO, Teruhisa, Tokyo 170-0003 (JP); SHINOHARA, Yuka, Tokyo 167-0051 (JP); KAWAMURA, Yoko, Tokyo 130-0002 (JP); NAGASE, Masaaki, Tokyo 192-0021 (JP)
(74) Representative: Dietz, Jörg-Reimar
(86) International application number: PCT/US1999/025555
(87) International publication number: WO 2000/027372

(56) References cited:
- EP-A- 0 271 983
- EP-A- 0 428 352
- EP-A1- 0 491 076
- US-A- 4 537 776
- US-A- 5 434 292
- US-A- 5 827 886

## Description

This invention relates to an anti-inflammatory and analgesic pharmaceutical preparation for external use having excellent percutaneous absorption and applicability.

Non-steroidal anti-inflammatory and analgesic agents have an excellent anti-inflammatory and analgesic activity and have been widely used in clinical field in the dosage form of pharmaceutical preparations for external use such as ointments, gel ointments, creams, lotions or sticking plasters, oral pharmaceutical preparations such as capsules or the like, suppositioies or injections.

It has been hitherto common that non-steroidal anti-inflammatory and analgesic agents (hereinafter referred to as NSAIDs) have been orally administered in the dosage form of capsules, tablets, etc. Although they when orally administered can show an excellent effect, they exhibit considerable side effects on absorption from the gastrointestinal tracts and, therefore, should be carefully handled in actual application. Accordingly, with an object of reducing the side effects such as gastrointestinal disorders by topical application for external use, there have been developed those techniques, for example, gels (Japanese Patent Kokai Application No. 161323/1981), creams (Japanese Patent Kokai Application Nos. 103811/1983 and 298526/1987), ointments (Japanese Patent Kokai Application No. 39616/1983), sticking plasters (Japanese Patent Kokai Application No. 250317/1989) and others. However, even in these inventions, there have remained the problems that, because of poor diffusion and transfer of the NSAIDs in a base phase of the sticking plasters, release of the drug is insufficient and that percutaneous absorption from ointments (gel bases) is not so good as expected.

In EP 0 428 352 A1, pharmaceutical compositions for external use comprising certain non-steroidal anti-inflammatory compounds (NSAIDs), such as piroxicam, naproxen and ibuprofen, and (C₄-C₃₀) fatty acids or alkyl or alkenyl esters thereof are disclosed. The latter work as enhancers of percutaneous penetration of said NSAIDs.

Accordingly, it is an object of this invention to provide a cutaneous pharmaceutical preparation for external use wherein the percutaneous absorption of the NSAIDs is improved.

Under such circumstances, the present inventors have made intensive studies and, as a result, have found that the percutaneous absorption of an NSAID is significantly improved by adding at least one percutaneous absorption promoting agent selected from the group consisting of oleic acid and oleyl alcohol to a specific water-soluble base.

More specifically, this invention relates to a pharmaceutical preparation for external use which comprises the NSAIDs and as a percutaneous absorption promoting agent oleic acid or oleyl alcohol in a pharmaceutically acceptable aqueous alcoholic solvent.

Thus, this invention relates to an anti-inflammatory and analgesic pharmaceutical composition for external use which comprises
- a non-steroidal anti-inflammatory and analgesic agent,
- 0.1 to 15 weight %, based on the total weight of the composition, of a skin penetration enhancer selected from the group consisting of oleic acid, oleyl alcohol and mixtures thereof,
- 0.5 to 5 weight %, based on the total weight of the composition, of menthol, and
- a pharmaceutically acceptable vehicle for the non-steroidal anti-inflammatory and analgesic agent and the skin penetration enhancer consisting of
   - 15 to 70 weight %, based on the total weight of the composition, of a monohydric saturated aliphatic alcohol containing 1 to 4 carbon atoms;
   - 0.1 to 30 weight %, based on the total weight of the composition, of a polyhydric alcohol selected from the group consisting of saturated aliphatic glycols containing 2 to 4 carbon atoms and glycerol; and
   - 10 to 60 weight %, based on the total weight of the composition, of water.

As the monohydric saturated aliphatic alcohol, there may be mentioned methanol, ethanol, n-propanol, isopropanol and the like, and ethanol and isopropanol may be particularly preferable.

As the saturated aliphatic glycol, there may be mentioned ethylene glycol, propylene glycol, 1,3-butylene glycol, isopropylene glycol (3-methyl-1,3-butanediol) and the like.

As the polyhydric alcohol, there may be mentioned said glycol and/or glycerol, and glycerol is particularly preferable.

A preferable blended proportion of such aqueous alcoholic solvent is 15-70% by weight, more preferably 30-65% by weight, for the monohydric saturated alcohol, 0.1-30% by weight, more preferably 0.5-15% by weight, for the polyhydric alcohol and 10-60% by weight, more preferably 20-50% by weight, for water. When the proportion of the monohydric saturated aliphatic alcohol is too low, solubility of the NSAIDs is lowered whereby turbidity appears and stability is lowered. The polyhydric alcohol, more preferably glycerol, may be preferably used at 5-30% by weight to the said monohydric saturated alcohol while satisfying said blended proportion.

It is essential in this invention to combine such specified aqueous alcoholic solvent with oleic acid and/or oleyl alcohol. These percutaneous absorption promoting agents may be used alone or in combination.

Oleic acid is preferred and cis-oleic acid is most preferred. A blended proportion of such percutaneous absorption promoting agents is 0.1-15% by weight based upon a total weight of the preparation for external use and, particularly, 0.5-10% by weight is preferable. If it is too small, a promoting effect on percutaneous absorption could not be accomplished.

Above about 15% promoting agent, further improvement in the absorption of the NSAID is not significant.

The active ingredient NSAIDs may be blended preferably at 0.1-10% by weight, particularly preferably at 0.5-5% by weight based upon a total weight of the preparation for external use.

It is necessary for the anti-inflammatory and analgesic preparation for external use to further incorporate therein menthol, especially 1-menthol. A blended proportion of menthol may be 0.5-5% by weight based upon a total weight of the preparation for external use.

The anti-inflammatory and analgesic pharmaceutical preparation of this invention may be preferably applied in the dosage form of liquids or gels for utilizing this invention more effectively. These dosage forms may use conventional bases and blending components in compliance with the desired dosage form. As the watersoluble polymers which may be commonly used as a gelling agent may be mentioned, for example, carboxyvinyl polymer, carboxymethylcellulose sodium, polyvinyl alcohol, methylcellulose, hydroxyethylcellulose, hydroxypropylcellulose, polyvinylpyrrolidone, hydroxypropylmethylcellulose, etc. such a gelling agent may be applied at 0.1-5% by weight based upon a total weight of the preparation for external use.

Also, there may be optionally added humectants, antiseptics, antioxidants, coloring agents, perfumes, thickening agents, etc., if required, to the anti-inflammatory and analgesic pharmaceutical preparation of this invention.

In accordance with this invention, there is provided a cutaneous pharmaceutical preparation for external use of the NSAIDs having an excellent percutaneous absorption property. Significantly, the dermatologic pharmaceutical preparation for external use of the NSAIDs having an excellent percutaneous absorption property can reduce side effects such as gastrointestinal, hepatic and renal disorders caused by continued oral administration and shows no pain which is a disadvantage in the case of invections and, therefore, a potent therapeutic effect can be expected to chronic articular rheumatish, peritendinitis, muscle pain, swelling and pain after woulds, etc.

Percutaneous absorption effect, pharmacological effect and stimulation to the skin by the anti-inflammatory and analgesic pharmaceutical preparation for external use according to this invention will be explained in greater detail by way of the following examples.

### Examples

The following formulated preparations were prepared by Examples 1-4 and Comparative Examples 1 and 2 according to a conventional method, while commercially available ketophenol-containing dermatologic drugs for external use were used in Comparative Examples 3-5.

### Example 1

| Lotions | |
|---|---|
| Ethanol | 56% by weight |
| Ketoprofen | 3% by weight |
| 1-Menthol | 3% by weight |
| Oleic Acid | 0.8% by weight |
| Oleyl Alcohol | 4.2% by weight |
| Glycerol | 6% by weight |
| Water | 27% by weight |

### Example 2

| Lotions | |
|---|---|
| Isopropanol | 56% by weight |
| Ketoprofen | 3% by weight |
| 1-Menthol | 3% by weight |
| Oleic Acid | 0.8% by weight |
| Oleyl Alcohol | 4.2% by weight |
| Glycerol | 6% by weight |
| Water | 27% by weight |

### Example 3

| Lotions | |
|---|---|
| Ethanol | 50% by weight |
| Ketoprofen | 3% by weight |
| 1-Menthol | 3% by weight |
| Oleic Acid | 4% by weight |
| Glycerol | 6% by weight |
| Water | 34% by weight |

### Example 4

| Gels | |
|---|---|
| Ethanol | 56% by weight |
| Ketoprofen | 3% by weight |
| 1-Menthol | 3% by weight |
| Oleic Acid | 0.8% by weight |
| Oleyl Alcohol | 0.2% by weight |
| Glycerol | 5.7% by weight |
| Hydroxypropylcellulose | 0.5% by weight |
| Diisopropanolamine | 0.4% by weight |
| Water | 29.8% by weight |

### Example 5

| Lotions | |
|---|---|
| Ethanol | 56% by weight |
| Indomethacin | 1% by weight |
| 1-Menthol | 3% by weight |
| Oleic Acid | 0.8% by weight |
| Oleyl Alcohol | 4.2% by weight |
| Glycerol | 6% by weight |
| Water | 27% by weight |

### Example 6

| Lotions | |
|---|---|
| Isopropanol | 56% by weight |
| Bufexamac | 1% by weight |
| 1-Menthol | 3% by weight |
| Oleic Acid | 0.8% by weight |
| Oleyl Alcohol | 4.2% by weight |
| Glycerol | 6% by weight |
| Water | 27% by weight |

### Example 7

| Gels | |
|---|---|
| Ethanol | 56.6% by weight |
| Ibuprofen | 2% by weight |
| 1-Menthol | 3% by weight |
| Oleic Acid | 0.8% by weight |
| Oleyl Alcohol | 0.2% by weight |
| Glycerol | 5.7% by weight |
| Hydroxypropylcellulose | 0.5% by weight |
| Diisopropanolamine | 0.4% by weight |
| Water | 29.8% by weight |

### Comparative Example 1

| Lotions | |
|---|---|
| Ethanol | 56% by weight |
| Ketoprofen | 3% by weight |
| 1-menthol | 3% by weight |
| Glycerol | 6% by weight |
| Water | 32% by weight |

### Comparative Example 2

| Lotions | |
|---|---|
| Ethanol | 71% by weight |
| Ketoprofen | 3% by weight |
| 1-Menthol | 3% by weight |
| Oleic Acid | 1% by weight |
| Oleyl Alcohol | 4% by weight |
| Glycerol | 18% by weight |

### Comparative Example 3 Lotions

Epatec A Lotion (Zeria-Nissan Chemical)

### Comparative Example 4

Epatec A Gel (Zeria-Nissan Chemical)

### Comparative Example 5 Indomethacin-containing gels

Vantelin Kowa Gel (Kowa)

### Comparative Example 7 Lotions

| Lotions | |
|---|---|
| Isopropanol | 56% by weight |
| Bufexamac | 1% by weight |
| 1-Menthol | 3% by weight |
| Glycerol | 6% by weight |
| Water | 34% by weight |

### Comparative Example 8 Lotions

| Lotions | |
|---|---|
| Ethanol | 71% by weight |
| Bufexamac | 1% by weight |
| 1-Menthol | 3% by weight |
| Oleic Acid | 1% by weight |
| Oleyl Alcohol | 4% by weight |
| Glycerol | 18% by weight |

### Test Examples for Evaluation

The NSAIDs containing pharmaceutical preparations made by Examples and Comparative Examples were tested for the evaluation of their percutaneous absorption effect, pharmacological effect and stimulation to skin.

### Test Example 1 Test on skin permeability in vitro

Hartley strain male guinea pigs 4 weeks of age were anesthetized with Nembutal by intraperitoneal injection and the hair on the back of the animals was clipped by hair clipper and then the skin was collected. Subcutaneous tissues were removed as much as possible from the collected skin without damaging the epidermis and the skin was cut into skin sheets of about 2 cm square. The sheets were placed in a diffusion cell of a Franz type having an effective diffusion area of 0.785 cm². The diffusion cell was kept at 37°C by a circulating water of constant temperature and the side of receptor of the cell was filled with 5 ml of an aqueous solution containing 5% of bovine serum albumin for receiving ketoprofen which was spanngly soluble in water. The skin was applied to the diffusion cell and allowed to stand for 2 hours until the system reached a stationary state and then 1 ml of a test solution was placed on the donor side of the cell. In order to prevent evaporation of the solution from the donor side, it was tightly sealed with parafilm. Thereafter, 100 µl each of the aqueous solutions at the receptor side was sampled with lapse of time. Acetonitrile (500 µl) was added to the collected sample to precipitate and remove the bovine serum albumin and the liberated ketoprofen was determined by a conventional method using HPLC. The measurement parameters for the HPLC were as defined below:

Column (for ketoprofen); STR ODS-II(I.D.4.6mm x L. 15 cm): Mobile phase; acetonitrile:100mM phosphate buffer=4:6 : Flow rate; 1 ml/min.: Column temperature; 25°C:Detection; 254nm.

Column (for indomethacin); STR ODS-II(I.D.4.6mm x L. 15 cm): Mobile phase; acetonitrile:10mM phosphate buffer=4:6 : Flow rate; 10 ml/min.: Column temperature; 25°C:Detection; 254nm.

Column (for bufexamac); STR ODS-II(I.D.4.6mm x L. 15 cm): Mobile phase; 2.5g of sodium 1-octanesulfonate and 0.6g of sodium ethylenetetraacetate were dissolved in 800 ml of water and to the solution were added 500ml of methanol, 500ml of acetonitrile and 8ml of glacial acetic acid: Flow rate; 1.0ml/min.: Column temperature; 25°C:Detection; 254nm.

Accumulated amounts of the NSAIDS which permeated through the skin to the receptor side after 12 hours from the initiation of the test are shown in Table 1.

**Table 1**

| Accumulated rate permeated through the skin | |
|---|---|
| **Ketoprofen 3%** | Permeated amount through the skin (µg/cm²) |
| Example 1 | 812 |
| Example 2 | 910 |
| Example 3 | 775 |
| Comparative Example 1 | 35 |
| Comparative Example 2 | 158 |
| Comparative Example 3 | 258 |

| | |
|---|---|
| **Indomethacin 1%** | Permeated amount through the skin (µg/cm²) |
| Example 5 | 320 |
| Comparative Example 5 | 91 |
| | |

| **Bufexamac 1%** | Permeated amount through the skin (µg/cm²) |
|---|---|
| Example 6 | 279 |
| Comparative Example 3 | 21 |

### Comparative Example 8

In the case of ketoprofen, lotions obtained by Examples 1-3 showed a better percutaneous absorption than that of lotion obtained by Comparative Example 1 containing neither oleic acid nor oleyl alcohol, lotion obtained by Comparative Example 2 containing no water and a competitive lotion product used in Comparative Example 3. Similar results were obtained in both cases of indomethacin and bufexamac. It is apparent from the above results that the anti-inflammatory and analgesic preparation for external use according to this invention can significantly promote the percutaneous absorption of the NSAIDs.

### Test Example 2 Test on pharmacological effect

### 2-1 Test on inhibition of edema induced by carrageenin

Anti-inflammatory action of the anti-inflammatory and analgesic preparation for external use according to this invention (Example 4) and that tof competitive preparations from another company (Comparative Examples 4 and 5) were evaluated by a test on inhibition of carrageenin-induced edema. Volume of right hind paw of rats of Wistar strain (male; eight weeks of age) was measured using a paw volume measuring device (MK-550; manufactured by Muromachi Kikai K.K.) and then the rats were divided into five groups (each group consisting of eight rats) so that the mean value of paw volumes in each of the group became approximately equal. Each preparation (50mg) was applied to the area around the site of right hind paw to which carrageenin is to be administered and, after 2 hours, the same amount was applied again to the same area. After 0.5 hours from the second application, the applied drug was removed using a gauge which was wetted with warm pure water and 0.08 ml of 1% carrageenin was subcutaneously administered immediately. After 2, 3 and 4 hours from the administration of carrageenin, paw volumes were measured and the swelling rate was calculated from the difference between the paw volumes before and after application of the preparation. Incidentally, after application of the preparation, the administered site was covered with a wrapping film (Salan Wrap; manufactured by Asahi Chemical Industries Co., Ltd.) and an expandable adhesive tape (Silky Tex; manufactured by ALCARE) was applied thereon to fix, so that the rats were no longer able to take the preparation orally. In control group, the same operations were conducted except for the application of the preparation.

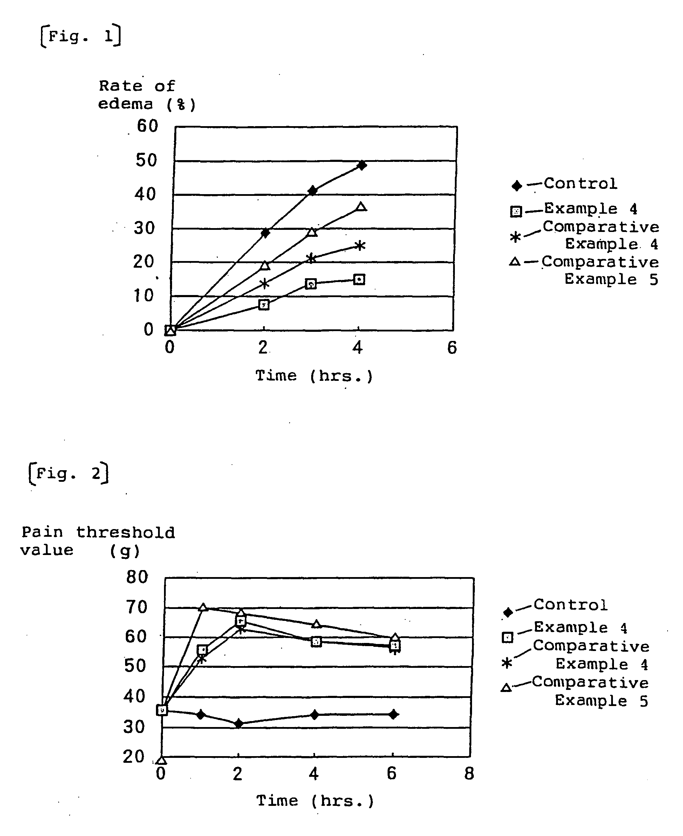

Test results are shown in Fig. 1. The preparation of Example 4 significantly inhibited the edema induced by subcutaneous administration of carrageenin from 2 to 4 hours since the induction. The swelling rate was lower than those of Comparative Examples 4 and 5 until 4 hours from the induction. The swelling rate of Example 4 at 4 hours from the induction was 14.8% (the inhibition rate to the control group was 70.2%) which was the lowest while the swelling rate (and the inhibiting rate in the same sense as above) of Comparative Examples 4 and 5 were 25.4% (47.7%) and 35.6% (26.7%), respectively.

### 2-2 Test on pain reaction of inflammatory paw induced by yeast (Randall and Selitto method)

Analgesic action of the anti-inflammatory and analgesic preparation for external use according to this invention (Example 4) and of competent products of another company (Comparative Examples 4 and 5) were evaluated by a test on pain reaction of inflammatory paw induced by yeast. Thus, 0.1 ml of a 20% yeast suspension was subcutaneously administered to right hind paw of Wistar strain (male; 5 weeks of age) so that inflammation was induced. After 2 hours, the pain threshold value of the inflammatory paw was measured using a threshold value measuring device of a compression type (MK-800; manufactured by Muromachi Kikai K.K.). Among the animals used, those wherein the pain threshold of the yeast-induced inflammatory paw was not more than 50% of the value before the infuction were selected and were divided into five groups (each group consisting of eight rats) so as to make the mean value of the pain threshold value in each group approximately equal. Each preparation (50mg) was applied around the site where the yeast was administered and the pain threshold value of the inflammatory paw after 1, 2, 4 and 8 hours from the administration was measured. Incidently, after application of the preparation, the administered site was covered with a wrapping film and an expandable adhesive tape was applied thereon to fix, so that the rats were no longer able to take the preparation orally. In control groups, the same operations were conducted except for the application of the preparation. The test results are shown in Figure 2.

As compared with the control group, the preparation of Example 4 significantly raised the pain threshold value, which had been lowered by subcutaneous administration of yeast, during the 1 to 6 hour penod following its application. The pain threshold value after one hour from the application showed the highest value (69.4g) and maintained the values of about 1.7-2.2-fold of those of the control until 6 hours. Comparative Examples 4 and 5 similarly raised the threshold values significantly until 6 hours, but the pain threshold values after one hour from the application were 51.9 and 65.6g for Comparative Examples 4 and 5, respectively, which were lower than the data by Example 4.

From the above results of the pharmacological tests, application of the product according to this invention to the inflammatory site showed quicker onset of the effect as compared with other anti-inflammatory and analgesic preparations and, additionally, the pharmaceutical effect was very good, while maintaining for 4 or 6 hours after application.

### Test Example 3 Evaluation of safety by application to human being

Safety of the anti-inflammatory and analgesic preparation for external use according to this invention (Example 4) and the competitive products of another company (Comparative Examples 4 and 5) in human healthy skin was investigated by an open patch test and a closed patch test by a closed sticking for 24 hours. The test was conducted by 18 healthy male adults (age: 20-24, 21.8 in average) and 18 females (age: 20-45; 25.8 in average). In an open patch test, a circle with 1 cm diamater was frawn on inner side of the upper arm of the persons to be tested, a preparation was uniformly applied within the circle using an applicator in accordance with a previously-fixed allotting table of the preparations and then determination was made after 20 minutes, 24 hours and 48 hours. In a closed patch test, a sample was permeated into a filter paper placed on a fin chamber and subjected to a closed adhesion to inner side of the upper arm using a Scanpor tap for 24 hours in accordance with a previously-fixed allotting table. Judgement was made after 60 minutes and also 24 hours from the removal of the sample. Both judgements were conducted in accordance with the standard regulated by the Patch Test Study Association of Japan as mentioned below.

| Criteria for judgement according to the Patch Test Study Association of Japan | |
|---|---|
| No reaction-----; | (-) |
| Slight red spots -----; | (\|) |
| Apparent red spots -----; | (+) |
| Red spots + Swelling; | (+ +) |
| Red spots + Swelling + Papules or Vesicles ; | (+ + +) |
| Big vesicles | -; (++++) |

In the case where apparent allergy reaction was noted, the corresponding score was marked with a circle.

Results of the adhesion test are shown in Table 2 (open patch test) and in Table 3 (closed patch test). In the open patch test, no reaction was noted on the skin in any of the preparations tested. In the closed patch test, one case of apparent red spots (+) was noted in Comparative Example 5 in the judgement of after 60 minutes from the removal and, in the judgement of after 24 hours from the removal, each one case of apparent red spots (+) was noted in Examples 4 and 5, while, in Example 4, only slight red spots (±) were noted. Table 3 shows stimulation indexes calculated from the results of the closed patch test. From this result, it is apparent that the preparations of Examples 4 and 5 are included within a category of allowable products. From the above, it can be concluded that the preparations of Examples 4 and 5 are a product having no problem in actual use, because, in the open patch test which is close to the situation of actual use, no positive reaction was noted at all while, in the closed patch test using a fin chamber, stimulation is apt to occur as compared with the actual use but the degree of the stimulation is similar to or less than that of the commercially available preparations.

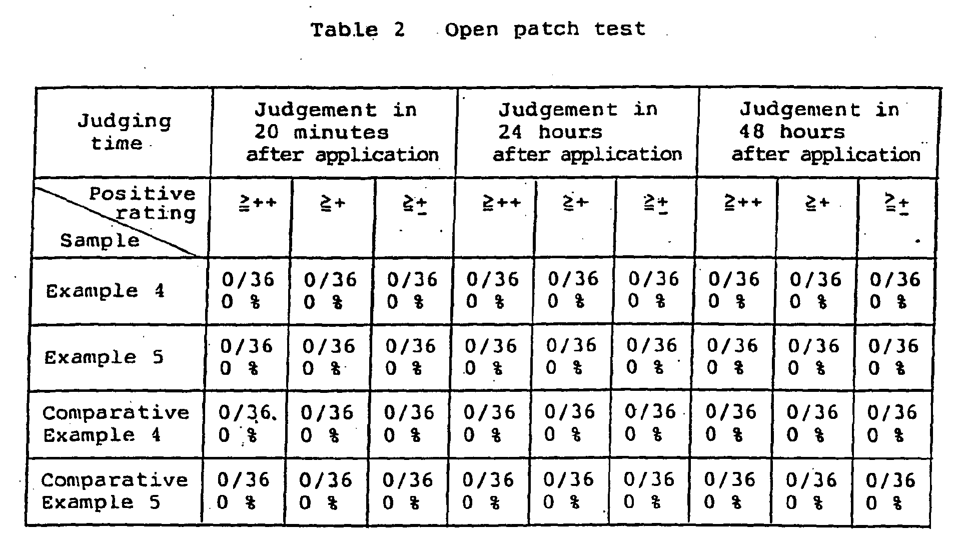

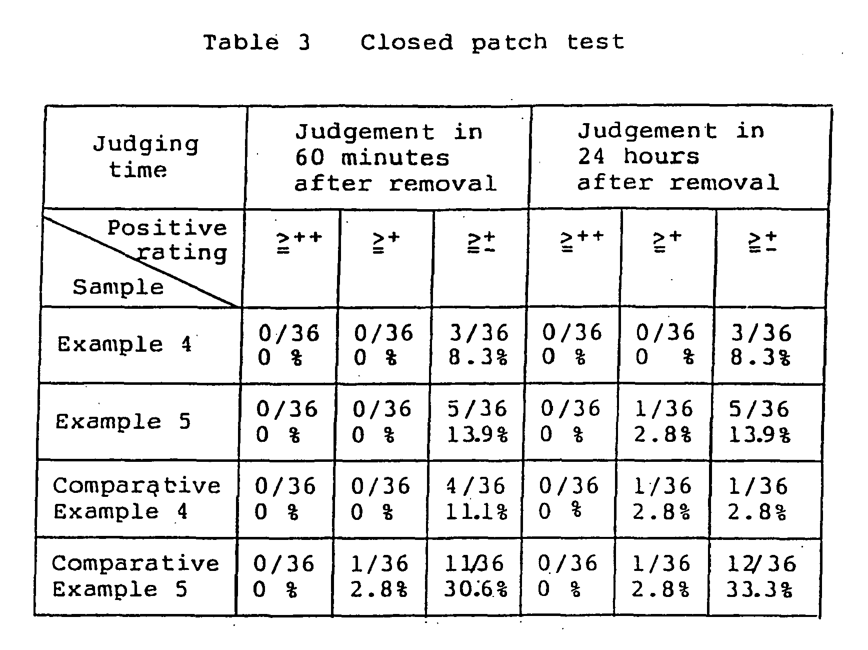

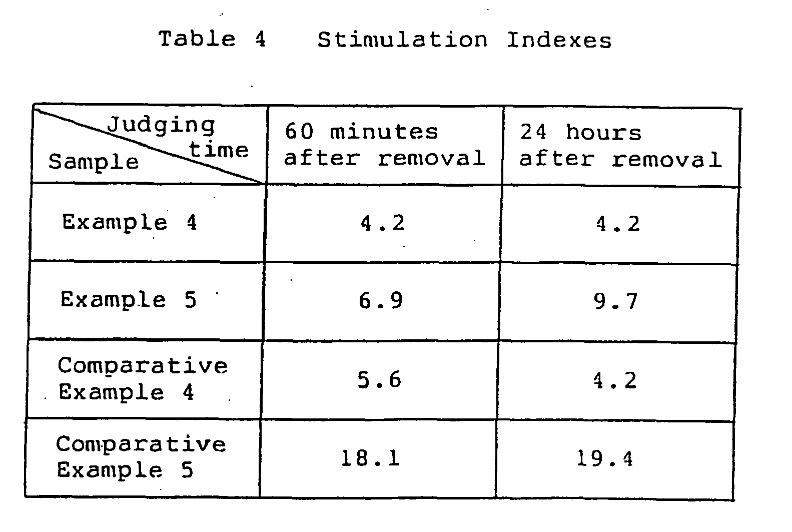

## Claims

1. An anti-inflammatory and analgesic pharmaceutical composition for external use which comprises
- a non-steroidal anti-inflammatory and analgesic agent,
- 0.1 to 15 weight %, based on the total weight of the composition, of a skin penetration enhancer selected from the group consisting of oleic acid, oleyl alcohol and mixtures thereof,
- 0.5 to 5 weight %, based on the total weight of the composition, of menthol, and
- a pharmaceutically acceptable vehicle for the non-steroidal anti-inflammatory and analgesic agent and the skin penetration enhancer consisting of
- 15 to 70 weight %, based on the total weight of the composition, of a monohydric saturated aliphatic alcohol containing 1 to 4 carbon atoms;
- 0.1 to 30 weight %, based on the total weight of the composition, of a polyhydric alcohol selected from the group consisting of saturated aliphatic glycols containing 2 to 4 carbon atoms and glycerol; and
- 10 to 60 weight %, based on the total weight of the composition, of water.

2. A composition according to claim 1, wherein the non-steroidal anti-inflammatory and analgesic agent is present in an amount of 0.1 to 10 weight %, based on the total weight of the composition.

3. A composition according to claim 1, wherein the non-steroidal anti-inflammatory and analgesic agent is present in an amount of 0.5 to 5 weight %, based on the total weight of the composition.

4. A composition according to any one of claims 1-3, wherein the skin penetration enhancer is present in an amount of 0.5 to 10 weight %, based on the total weight of the composition.

5. A composition according to any one of claims 1-4, which further comprises a gelling agent.

6. A composition according to claim 5, wherein the gelling agent is selected from the group consisting of carboxyvinyl polymer, sodium carboxymethyl cellulose, polyvinyl alcohol, methyl cellulose, hydroxyethyl cellulose, hydroxypropyl cellulose, polyvinyl pyrrolidone and hydroxypropyl methylcellulose.

7. A composition according to claim 5 or claim 6, wherein the gelling agent is present in an amount of 0.1 to 5 weight %, based on the total weight of the composition.

8. A composition according to any one of claims 1-7, wherein the non-steroidal anti-inflammatory and analgesic agent is selected from the group consisting of ketoprofen, indomethacin, bufexamac, ibuprofen and piroxicam.

9. A composition according to any one of claims 1-8, wherein the skin penetration enhancer is oleic acid.

10. A composition according to any one of claims 1-8, wherein the skin penetration enhancer is a mixture of oleic acid and oleyl alcohol.

11. A composition according to any one of claims 1-10, wherein the monohydric saturated aliphatic alcohol containing 1 to 4 carbon atoms is selected from the group consisting of ethanol and isopropanol.

12. A composition according to any one of claims 1-11, wherein the polyhydric alcohol is glycerol.

## Patentansprüche

1. Entzündungshemmende und analgetische pharmazeutische Zusammensetzung zur äußeren Verwendung, umfassend
- ein nicht steroidales entzündungshemmendes und analgetisches Mittel,
- 0,1 bis 15 Gew.%, bezogen auf das Gesamtgewicht der Zusammensetzung, eines Hautpenetrationsenhancers, ausgewählt aus der Gruppe, bestehend aus Ölsäure, Oleylalkohol und Gemischen davon,
- 0,5 bis 5 Gew.-%, bezogen auf das Gesamtgewicht der Zusammensetzung, Menthol, und
- ein pharmazeutisch akzeptables Vehikel für das nicht steroidale entzündungshemmende und analgetische Mittel und den Hautpenetrationsenhancer, bestehend aus
- 15 bis 70 Gew.-%, bezogen auf das Gesamtgewicht der Zusammensetzung, eines einwertigen, gesättigten, aliphatischen Alkohols, enthaltend 1 bis 4 Kohlenstoffatome;
- 0,1 bis 30 Gew.-%, bezogen auf das Gesamtgewicht der Zusammensetzung, eines mehrwertigen Alkohols, ausgewählt aus der Gruppe, bestehend aus gesättigten aliphatishen Glycolen, enthaltend 2 bis 4 Kohlenstoffatome, und Glycerol; und
- 10 bis 60 Gew.-%, bezogen auf das Gesamtgewicht der Zusammensetzung, Wasser,

2. Zusammensetzung nach Anspruch 1, wobei das nicht steroidale entzündungshemmende und analgetische Mittel in einer Menge von 0,1 bis 10 Gew.-%, bezogen auf das Gesamtgewicht der Zusammensetzung, vorliegt.

3. Zusammensetzung nach Anspruch 1, wobei das nicht steroidale entzündungshemmende und analgetische Mittel in einer Menge von 0,5 bis 5 Gew.-%, bezogen auf das Gesamtgewicht der Zusammensetzung, vorliegt.

4. Zusammensetzung nach einem der Ansprüche 1 bis 3, wobei der Hautpenetrationsenhancer in einer Menge von 0,5 bis 10 Gew.-%, bezogen auf das Gesamtgewicht der Zusammensetzung, vorliegt.

5. Zusammensetzung nach einem der Ansprüche 1 bis 4, ferner umfassend ein Geliermittel.

6. Zusammensetzung nach Anspruch 5, wobei das Geliermittel aus der Gruppe ausgewählt ist bestehend aus Carboxyvinylpolymer, Natriumcarboxymethylcellulose, Polyvinylalkohol, Methylcellulose, Hydroxyethylcellulose, Hydroxypropylcellulose, Polyvinylpyrrolidon und Hydroxypropylmethylcellulose.

7. Zusammensetzung nach Anspruch 5 oder Anspruch 6, wobei das Geliermittel in einer Menge von 0,1 bis 5 Gew.-%, bezogen auf das Gesamtgewicht der Zusammensetzung, vorliegt.

8. Zusammensetzung nach einem der Ansprüche 1 bis 7. wobei das nicht steroidale entzündungshemmende und analgetische Mittel aus der Gruppe ausgewählt ist, bestehend aus Ketoprofen, Indomethacin, Bufexamac, Ibuprofen und Piroxicam.

9. Zusammensetzung nach einem der Ansprüche 1 bis 8, wobei der Hautpenetrationsenhancer Ölsäure ist.

10. Zusammensetzung nach einem der Ansprüche 1 bis 8, wobei der Hautpenetrationsenhancer ein Gemisch aus Ölsäure und Oleylalkohol ist.

11. Zusammensetzung nach einem der Ansprüche 1 bis 10, wobei der einwertige, gesättigte, aliphatische Alkohol, enthaltend 1 bis 4 Kohlenstoffatome, aus der Gruppe ausgewählt ist, bestehend aus Ethanol und Isopropanol.

12. Zusammensetzung nach einem der Ansprüche 1 bis 11, wobei der mehrwertige Alkohol Glycerol ist.

## Revendications

1. Composition pharmaceutique anti-inflammatoire et analgésique destinée à une utilisation par voie externe qui comprend :
- un agent anti-inflammatoire et analgésique non-stéroïdien,
- de 0,1 à 15% en poids sur base du poids total due la composition, d'un agent favorisant la pénétration à travers la peau sélectionné à partir du groupe composé de l'acide oléique, de l'alcool oléilique et de mélanges de ceux-ci,
- de 0,5 à 5% en poids sur base du poids total de la composition, de menthol, et
- un véhicule pharmaceutiquement acceptable pour l'agent anti-inflammatoire et analgésique non-stéroïdien et l'agent favorisant la pénétration à travers la peau composé :
- de 15 à 70% en poids sur base du poids total de la composition, d'un alcool aliphatique monohydrique saturé contenant de 1 à 4 atomes de carbone ;
- de 0,1 à 30% en poids sur base du poids total de la composition, d'un alcool polyhydrique sélectionné à partir du groupe composé de glycols aliphatiques saturés contenant de 2 à 4 atomes de carbone et du gycérol ; et
- de 10 à 60% en poids sur base du poids total de la composition, d'eau

2. Composition selon la revendication 1, **caractérisée en ce que** l'agent anti-inflammatoire et analgésique non-stéroïdien est présent selon une quantité comprise dans la gamme allant de 0,1 à 10% en poids sur base du poids total de la composition.

3. Composition selon la revendication 1, **caractérisée en ce que** l'agent anti-inflammatoire et analgésique non-stéroïdien est présent selon une quantité comprise dans la gamme allant de 0,5 à 5% en poids sur base du poids total de la composition.

4. Composition selon l'une quelconque des revendications 1-3, **caractérisée en ce que** l'agent favorisant la pénétration à travers la peau est présent selon une quantité comprise dans la gamme allant de 0,5 à 10% en poids sur base du poids total de la composition.

5. Composition selon l'une quelconque des revendications 1-4 comprenant également un agent gélifiant.

6. Composition selon la revendication 5, **caractérisée en ce que** l'agent gélifiant est sélectionné à partir du groupe composé du polymère carboxyvinylique, carboxyméthylcellulose de sodium, alcool polyvinylique, méthylcellulose, hydroxyéthylcellulose, hydroxypropylcellulose, polyvinylpyrrolidone, hydroxypropylméthylcellulose.

7. Composition selon la revendication 5 ou 6, **caractérisée en ce que** l'agent gélifiant est présent selon une quantité comprise dans la gamme allant de 0.1 à 5% en poids sur base du poids total de la composition.

8. Composition selon l'une quelconque des revendications 1-7, **caractérisée en ce que** l'agent anti-inflammatoire et analgésique non-stéroïdien est sélectionné à partir du groupe composé de kétoprofène, indométhacine, bufexamac, ibuprofène et piroxicam.

9. Composition selon l'une quelconque des revendications 1-8, **caractérisée en ce que** l'agent favorisant la pénétration à travers la peau est l'acide oléique.

10. Composition selon l'une quelconque des revendications 1-8, **caractérisée en ce que** l'agent favorisant la pénétration à travers la peau est un mélange d'acide oléique et d'un alcool oléilique.

11. Composition selon l'une quelconque des revendications 1-10, **caractérisée en ce que** l'alcool aliphatique monohydrique saturé contenant de 1 à 4 atomes de carbone est sélectionné à partir du groupe composé d'éthanol et d'isopropanol.

12. Composition selon l'une quelconque des revendications 1-11, **caractérisée en ce que** l'alcool polyhydrique est du glycérol.
